Europäisches Patentamt

**European Patent Office**

Office européen des brevets

(11) Publication number: **0 073 675**
**B1**

(12) **EUROPEAN PATENT SPECIFICATION**

(45) Date of publication of patent specification: **04.12.85**

(51) Int. Cl.⁴: **C 12 M 1/36, C 12 M 1/04**

(21) Application number: **82304566.1**

(22) Date of filing: **31.08.82**

(54) Continuous fermentation apparatus and process.

(30) Priority: **31.08.81 US 297760**

(43) Date of publication of application:
**09.03.83 Bulletin 83/10**

(45) Publication of the grant of the patent:
**04.12.85 Bulletin 85/49**

(84) Designated Contracting States:
**DE FR GB NL**

(56) References cited:
AT-B- 339 848
DE-A-2 127 747
DE-C- 271 250
FR-A-2 392 608
FR-A-2 406 663
FR-A-2 450 567
GB-A-2 034 750
GB-A-2 077 712
US-A-3 723 255
US-A-3 986 932

BIOTECHNOLOGY AND BIOENGINEERING,
vol.23, no.2, February 1981, New York (US), A.
VERES et al.: "Automated fermentation
equipment I.Program-controlled fermentor",
pages 391-404

(73) Proprietor: **Corning Glass Works**
**Houghton Park**
**Corning New York 14831 (US)**

(72) Inventor: **Gregory, Jerry Lee**
**2008 Skylab Terrace**
**Martinsburg West Virginia (US)**
Inventor: **Pitcher, Wayne Harold, Jr.**
**302 Green Valley Drive**
**Big Flats New York (US)**
Inventor: **Sharma, Bhavender Paul**
**Box 246, R.D.1 1205 State Road**
**Lindley New York (US)**

(74) Representative: **Smith, Sydney**
**Elkington and Fife High Holborn House 52/54**
**High Holborn**
**London WC1V 6SH (GB)**

Courier Press, Leamington Spa, England.

# 0 073 675

**Description**

This invention relates to a continuous fermentation apparatus and process. Fermentation is the conversion of organic compounds to biomass and/or other organic compounds by living cells.

In the past, the microbes used in a fermentation process have been mixed into the liquid medium within the fermentation tank. Apparatus in which fermentation may be conducted on both a batch and a continuous basis are known. An example is the "Bioflow" fermenter produced by New Brunswick Scientific Company (Edison, New Jersey, U.S.A.). In this apparatus, the fermenter tank is filled with sterile nutrient medium or broth. The fermenter contents then are inoculated with the desired microorganism for the particular fermentation. The microorganisms are typically prepared by culturing in, for example, an Erlenmeyer flask outside of the fermenter apparatus. The resulting inoculum is then transferred to the fermenter tank containing nutrient medium. Such tank then is ready for batch fermentation.

Continuous operation of a fermenter apparatus involves the inoculation of a fermenter tank containing nutrient broth, as described above. The microorganisms so inoculated are permitted to grow within the fermenter tank until the late logarithmic phase in the microorganism growth cycle has been reached. Nutrient broth or feed is then pumped to the fermenter tank at a desired flow rate. Agitation, and aeration in the case of aerobic fermentation of the fermenter contents are also initiated. After the system within the fermenter tank reaches a steady state, output flow is begun.

In recent years, attention has turned to improving fermentation efficiencies by immobilizing the microbes on a support material or carrier within the fermenter tank. Typically, microorganisms are adsorbed on or chemically coupled to a carrier which is packed into a column in which fermentation is conducted. As is well known, such carriers may be organic or inorganic and encompass a broad range of materials.

Over the years, various recycling embodiments of fermentation processes have been described, the most pertinent of which are summarized below.

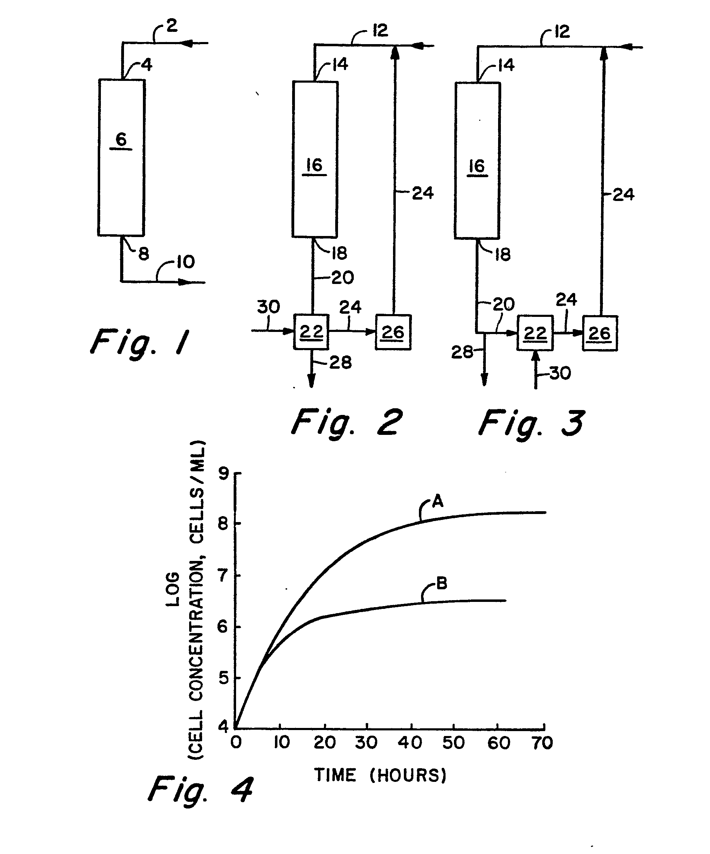

United States Patent No. 2,658,034 discloses a sewage treatment process which may involve recycling a portion of the product as an inoculum to an aeration tank, through which the raw sewage passes on its way to a biological oxidation tank. Of particular interest are schematic Figures 2 and 3. The ratio of recycle stream to feed is apparently of the order of about 0.2. Recycling serves the dual purpose of inoculating the feed with active bacteria and giving a slight downward adjustment in feed pH.

United States Patent No. 4,169,010 relates to a continuous fermentation process for the production of single cell protein in which lean ferment is recycled through an absorber in contact with oxygen-containing off-gases from the fermentation tank. Lean ferment is obtained upon removal of the single-cell protein materials from the fermentation medium. Such recycle permits recovery of at least a portion of the oxygen contained in the off-gases.

United States Patent No. 3,705,082 discloses a method and apparatus for cultivating microorganisms wherein partially deaerated broth is recycled to the cultivation zone, thereby improving pumping efficiency.

United States Patent No. 3,833,477 relates to aerobic fermentation of hydrocarbons by a process which involves recycling an oxygen-containing gaseous mixture (originally introduced as the source of oxygen) to the fermentation reactor after enriching such mixture with oxygen and simultaneously removing, at least partially, at least one impurity contained in such mixture.

United States Patent No. 4,032,407 discloses a tapered fluidized bed reactor. Example 1 at column 4 of this reference mentions a reactor which uses anthracite coal as the packing material for the bacteria and suggests that a portion of the waste stream from the reactor may be recycled (see column 4, lines 56 to 58). This reference is of interest for disclosing an immobilized microbe reactor. However, from the Figure, there appears to be no aeration chamber from which the waste stream is recycled to a reaction zone. Recycle of a portion of the reactor effluent apparently serves only to dilute the feed stream in cases where available oxygen becomes limiting.

United States Patent No. 4,048,017 relates to a continuous fermentation apparatus in which a nutrient, such as a carbon source, is supplied in an amount which is stoichiometrically proportional to the oxygen that is supplied so that oxygen does not become the limiting nutrient in the process.

United States Patent No. 4,225,430 teaches recycle in a denitrofying process employing bacteria in a packed bed for the continuous removal of excess biomass accumulation. The ratio of recycle stream to feed ranges from 2 to 200. Such recycle, however, serves only to provide a reactor velocity which is sufficient continuously to remove some of the biomass from the reactor bed particles, thereby establishing a balance between bacterial growth rate and biomass wash-off.

United States Patent No. 4,009,099 discloses a process for removing ammonia nitrogen from waste water wherein waste water and oxygen are passed continuously through a fluidized bed of solid particulate carrier cultured with nitrifying microorganisms. Optionally, the effluent gases from the fluidized bed may be recycled into waste water, or the waste may be recycled to enhance greater oxygen adsorption. Figure 2, however, shows recycled effluent being admixed with waste water prior to oxygenation of the reactor influent. The recycling of selected portions of the effluent is said to serve the following purposes: (1) to promote microorganism growth during seeding; (2) to maintain uniform flow; (3) to dilute the waste water; (4) to provide more oxygen to the waste water; and (5) to permit the additional removal of ammonia remaining in the effluent (see column 10, lines 22—32). There is no indication, however, that such recycle ever becomes a major portion of the reactor influent.

United States Patent No. 4,009,105 discloses a method and apparatus for biologically removing bio-chemical oxygen demand from waste water which are similar to the disclosure of U.S. Patent No. 4,009,009. In this case, however, selected portions of the effluent are recycled to the influent waste water after oxygenation of the waste water.

GB—A—2,034,750 relates to a fermentation process e.g. yeast in beer, in a fermenting vessel, which comprises the step of drawing off a fermenting broth of microorganisms in a substrate liquid, separating fermenting microorganisms in the broth recycling them separately, while the substrate liquid is pressurised, saturated with a nutrient gas and then returned to the fermenting vessel. An aerobic fermenter recycles a portion of the fermentation broth in order to remove excess micro-organism growth. The recycled substrate, without organisms after passing through a separator, is oxygenated before being returned. The recycling of a feed in a free-floating fermentation allows the use of a high recycle rate, but concomitantly requires the use of a separator in the recycle loop.

GB—A 2,007,712 relates to a process for the bio-treatment of a liquid mass by means of contact with a biomass, which process comprises the following stages:

(a) Oxygenation of the liquid mass, prior to contact with the biomass, by emulsification through the addition of oxygenating gas, in such a way that the velocity of the liquid flow sucks in the said gas;

(b) Fine distribution of the liquid mass;

(c) Contact with the biomass;

(d) Separation of at least part of the gas sucked in by the liquid mass;

(e) Recirculation of the liquid mass to stage (a).

An unknown portion of effluent is recycled in an aerobic bioreactor. Before re-entering the reactor, but after entering the inlet feed means, the recycled effluent is converted to an emulsion by passage through a venturi. An oxygenating emulsion is used to supply oxygen to immobilized microorganisms. The emulsion relies on gravity, not a positive flow, to pass through the reactor before being pumped to the reactor top.

The prior art is in no way suggestive of the present improved apparatus and process.

Experimental studies have shown that continuous fermentation processes may be conducted in a significantly more efficient manner in an immobilized microbe reactor if certain technical problems are solved. For example, among such problems are those associated with bringing sufficient nutrients into contact with the microorganisms, particularly when one or more nutrients are sparingly soluble in the aqueous fermentation medium. Because readily soluble nutrients tend to be available in excess, the sparingly soluble nutrients become productively-limiting.

By way of illustration, the classic productivity-limiting nutrient in aerobic fermentation processes is oxygen. In the past, attempts have been made to overcome oxygen deficiencies, for example, by the use of trickling filter-type fermenters and, in one case, by bubbling air through a fluidized bed of microbial pellets. Indeed, aeration of the fermentation tank contents is commonplace in known aerobic fermentation procedures. Nevertheless, satisfying the oxygen demand of a rapidly growing aerobic microbial cell population may be a problem with a conventional single-pass reactor system.

An object of the present invention is to provide an improved continuous immobilized microbe fermenter system and process characterised by an increased supply of a fermentation productivity-limiting nutrient.

A further object of the present invention is to improve the nutrient supply to microorganisms in a continuous immobilized microbe fermenter wherein a sparingly soluble nutrient, such as oxygen, is the productivity-limiting ingredient.

Accordingly, the present invention provides a continuous fermentation apparatus characterised in that it comprises:

(A) an immobilized microbe reactor having an inlet and an outlet;

(B) a nutrient supply means;

(C) a first means connected to the said inlet for supplying feed thereto;

(D) a second means connecting the said outlet to the said nutrient supply means;

(E) a third means connecting the said nutrient supply means to the said first means and having a flow regulation means suitable for recycling a portion of the effluent from the said outlet to the said first means at a recycle ratio of nutrient-enriched effluent to feed of from 30:1 to 120:1; and

(F) a fourth means for withdrawing from the said continuous fermentation apparatus that portion of effluent from the said outlet which is not recycled to the said first means, which fourth means is connected to the said outlet, the said second means, the said nutrient supply means or the said third means at a point from the said nutrient supply means to and including the said flow regulation means.

The present invention also provides a continuous fermentation process characterised in that it comprises:

(A) continuously passing a feed through an immobilized microbe reactor;

(B) continuously adding at least one productivity-limiting nutrient to at least a portion of the effluent from the said reactor;

(C) continuously recycling at least a portion of the nutrient-enriched effluent from (B) through the said reactor at a recycle ratio of nutrient-enriched effluent to feed of from 30:1 to 120:1, with the proviso that the

# 0 073 675

volume ratio of effluent from the said reactor to recycled nutrient-enriched effluent is greater than unity; and

(D) continuously withdrawing for disposal or further processing that portion of effluent from the said reactor which is not recycled.

In certain embodiments of the present invention, the productivity-limiting nutrient is a gas which is sparingly soluble in the feed. In preferred embodiments, the said nutrient is oxygen.

Referring to the accompanying illustrative drawings:

Figure 1 is a schematic diagram of one embodiment of a conventional single-pass immobilized microbe reactor.

Figure 2 is a schematic diagram of one embodiment of the continuous fermentation apparatus according to the present invention.

Figure 3 is a schematic diagram of another embodiment of the continuous fermentation apparatus according to the present invention.

Figure 4 is a graph which shows cell generation versus time with immobilized *Escherichia coli* in both a continuous fermentation apparatus according to the present invention and a conventional single-pass reactor.

As mentioned above, the term "fermentation" is used herein to mean the conversion of organic compounds to biomass and/or other organic compounds by living cells. In general, such cells include various cells capable of being sustained in a viable condition in an immobilized state. Thus, such cells may be microorganisms, such as algae, bacteria, blue-green algae, fungi and yeasts, as well as other plant and animal cells, such as protozoa and cells obtained or derived from higher plants and animals.

Such conversion may be aerobic, anaerobic or some combination thereof. The purpose of such conversion, however, is not critical to the present invention. Thus, such conversion may be directed to, for example, the cultivation of microbial or other cells; the production of single-cell protein; the production of useful products, such as antibiotics, amino acids or carboxylic acids; or waste processing, either with or without the production of useful products, such as ethanol and methane.

From the above, it should be clear that the term "immobilized microbe reactor" is intended to include cells other than microbial cells. As used herein, then, the term "microbe" may be and often is used in a generic sense to encompass various cells capable of being sustained in a viable condition in an immobilized state.

The term "recycle ratio", referred to in more detail hereinafter, is used herein to mean the ratio of the recycle flow rate of nutrient enriched effluent to feed flow rate, with each flow being expressed as a volume per unit time.

Finally, the term "composite" is used herein to mean the carrier with microorganisms attached or bound thereto.

Turning now to the accompanying drawings, Figure 1 is a schematic diagram of an embodiment of a conventional single-pass immobilized microbe reactor. Feed is introduced into immobilized microbe reactor 6 at inlet 4 through feed supply means 2. Effluent emerges from reactor 6 at outlet 8 and is removed from the apparatus by effluent withdrawal means 10.

Referring to Figure 2, which is a schematic diagram of one embodiment of a continuous fermentation apparatus according to the present invention (often referred to hereinafter as the recycle apparatus), feed is introduced into immobilized microbe reactor 16 at inlet 14 through feed supply means 12. Effluent emerges from reactor 16 at outlet 18 and is carried by connecting means 20, such as a conduit or pipe, to nutrient supply means 22, in which the effluent and a sparingly soluble productivity-limiting nutrient are admixed to give a nutrient-enriched effluent. A portion of the nutrient-enriched effluent is then carried to inlet 14 by connecting means 24, such as a conduit or pipe, which has associated therewith flow regulation means 26 which is suitable for recycling the nutrient-enriched effluent through reactor 16 at a recycle ratio of from 30:1 to 120:1. Nutrient supply means 22 is fitted with withdrawal means 28, such as a conduit or pipe, in order to remove from the fermentation apparatus that portion of nutrient-enriched effluent which is not recycled through reactor 16. Depending upon the nature of the productivity-limiting nutrient, nutrient supply means 22 is optionally fitted with nutrient introduction means 30, such as a conduit or pipe. For example, when the productivity-limiting nutrient is a gas, such as oxygen, nutrient supply means 22 is a chamber for contacting a liquid with a gas, such as an aeration chamber, having a continuous supply of air or other free oxygen containing gas provided through nutrient introduction means 30.

In the embodiment shown in Figure 2, productivity-limiting nutrient is added to all of the effluent from the reactor. Thus, a portion of such nutrient is in effect wasted since not all of the nutrient-enriched effluent is recycled through the reactor. Consequently, a more efficient utilization of the productivity-limiting nutrient involves admixing such nutrient with only that portion of effluent which is to be recycled through the reactor. Such an embodiment, which is preferred, is shown in Figure 3.

Figure 3, of course, differs from Figure 2, only in the location of withdrawal means 28. In Figure 3, withdrawal means 28 is fitted to connecting means 20, rather than to nutrient supply means 22 as shown in Figure 2.

For convenience, the illustrative discussion which follows focuses primarily on the problem of providing sufficient oxygen for aerobic fermentation processes which utilize immobilized microbe reactors.

4

**0 073 675**

It should be understood that various sparingly soluble nutrients whether gaseous, solid, or liquid may be employed in accordance with the present invention.

Using oxygen in the form of an oxygen-containing gas as a representative sparingly-soluble nutrient, the recycling of a portion of the aerated effluent results in higher output and higher cell concentrations within the fermenter because of greatly improved oxygen contact with the immobilized cells for a given feed rate. Thus, in accordance with the present apparatus and process, at least a portion of the effluent from the immobilized microbe reactor is aerated in an aeration chamber. Such chamber is represented in Figures 2 and 3 as nutrient supply means 22 which is in turn fitted with a conduit or pipe through which the oxygen is introduced into the aeration chamber, i.e. nutrient introduction means 30.

In the embodiment illustrated in Figure 2, all of the effluent from the reactor enters the aeration chamber where continuous aeration takes place. A portion of the aerated effluent is then recycled through the reactor at a recycle ratio of nutrient-enriched effluent to feed of from 30:1 to 120:1. The remainder of the aerated effluent, i.e. that portion of aerated effluent which is not yet recycled, is continuously withdrawn from the aeration chamber, e.g. via withdrawal means 28.

In the embodiment illustrated in Figure 3, only a portion of the effluent from the reactor enters the aeration chamber, with the remainder of the effluent bypassing the aeration chamber. In this embodiment, all of the effluent which is aerated is recycled through the reactor.

Although not illustrated, it will be apparent to those skilled in the art that the aeration chamber may be fitted with a recycle means which would permit recycling excess oxygen through the aeration chamber.

In general, the preparation of the immobilized microbe reactor is accomplished in accordance with well known procedures. By way of illustration, the preparation of a typical immobilized microbe reactor is as follows: A culture of microorganisms, such as *E. coli* K12 or *B. subtilis*, is grown in a shake flask under aseptic conditions with agitation to allow for sufficient oxygen transfer and mixing of the microbes with nutrients. The resulting inoculum is then adsorbed on or chemically coupled to the packing material or carrier of the reactor. Sterile nutrient medium, such as a nutrient broth, is prepared by autoclaving. The sterile nutrient medium is then passed through the reactor for from three to five days. Effluent from the reactor is sampled periodically and effluent cell concentration is estimated by, for example, the viable plate count method or turbidity measurement. After attaining a steady state cell concentration within the reactor, the continuous fermentation process is started by activating the feed supply, aeration, effluent withdrawal and recycling as described above.

As indicated above, the microorganisms are typically coupled or attached to the carrier particles outside of the reactor. The resulting microbe-carrier composite is then charged to the reactor prior to the start-up of the continuous fermentation process. Thus, the carrier particles with which the reactor is charged are typically coated and/or impregnated with the desired microbial cells.

However, other procedures are known and may be used. For example, the reactor may be charged with the selected carrier particles and typical sterilization procedures carried out, such as autoclaving or chemical treatments with, for example, ethylene oxide. A suspension of the desired microbial cells in sterile nutrient medium is then continuously recirculated through the reactor for such a time and under conditions sufficient to permit the cells to attach or couple to the carrier.

The nature of the reactor packing or carrier is not critical to either the apparatus or process according to the present invention. Thus, the carrier may be organic or inorganic, porous or non-porous and in any desired shape or form. The carrier may be particulate in nature, varying from a finely-divided powder to a coarse granular material, or the carrier may be a continuous shaped article, such as a flat or curved sheet or pellet, or a three-dimensional article, such as a rectangular or cylindrical tube or a complex monolith. As a practical matter, the carrier will most often be particulate and relatively finely-divided.

Examples of suitable organic carriers include polyesters, such as poly(ethylene terephthalate); polyamides, such as nylon 6 and nylon 6,6; polyacrylates; polymethacrylates; agarose and dextran gels, typically strengthened by cross-linking; polyolefins, such as polyethylene, polypropylene, polybutene and polybutadiene; polystyrene, with or without cross-linking; poly(vinyl chloride); and poly(vinylidine chloride).

The more suitable inorganic carriers may be classified as siliceous or non-siliceous metal oxides. Examples of siliceous carriers include glass, silica, wollastonite, bentonite and cordierite. Examples of non-siliceous metal oxides include, alumina, spinel, apatite, nickel oxide, titania and zirconia. The preferred carriers are inorganic in nature, with siliceous materials being more preferred. Preferably, the carrier will be porous in order to provide a greater biomass per unit volume of carrier.

The method of immobilization of the microbes is not critical to the present invention. In general, the microbes may be immobilized by various known means which may vary from simple adsorption to chemical coupling. As a practical matter, however, simple adsorption is preferred for reasons of convenience and economy. Particularly useful adsorption procedures are those described in U.S. Patents Nos. 4,149,936, 4,149,937 and 4,153,510.

Reactor design is also not critical, provided only that the carrier is retained in the reactor. Thus, where the carrier is particulate, a packed-bed or fluidized-bed configuration may be employed, depending upon the rate and direction of flow through the reactor. The reactor may comprise a single vessel or column or a plurality of vessels or columns. In the latter case, such plurality of vessels may be arranged in series or in parallel. Thus, reactor design may be selected at will from the prior art, with modification thereof to

5

accommodate the recycle function provided in accordance with the present invention. In addition, there are no limits on the volumes of the reactors employed in accordance with the present invention. For example, very large reactors of 380 litre (100 U.S. gallon) capacity or more may be used, as well as very small reactors, such as those having a capacity of 45 ml or less.

As stated above, the recycle ratio must be from 30:1 to 120:1, although the recycle ratio employed in the given case will depend on various factors, such as the desired cell or product concentration in the effluent, the concentrations of readily soluble nutrients in the feed, oxygen saturation levels in the effluent and feed (which in turn are dependent upon, for example, temperature, pressure, feed and effluent compositions) and reactor back-pressures associated with flow rates through the reactor. For a particular application, one factor may be more important than others. For example, if an aerobic fermentation is to be carried out where high product concentration is important, a very high recycle ratio may be optimal. For example, if 100 times as much oxygen is required as may be dissolved in the feed stream, a recycle ratio of 99:1 may be required. Since such nutrients as carbohydrates are normally much more soluble than oxygen, it may frequently be desired to use rather high recycle ratios.

Of course, the residence time required in the reactor will be dictated by the productivity rate of the immobilized microbes. However, the residence time is a function of both the feed rate and the recycle rate. That is, for a given feed rate, the residence time is inversely proportional to the recycle ratio. Thus, the optimization of the residence time will generally require a balance between the feed rate and the recycle ratio. Stated differently, the optimization of the residence time may require reducing the feed rate and recycle rate to maintain the desired recycle ratio without deviating significantly from the desired or optimum residence time.

For a given fermentation process carried out in accordance with the present invention, some routine experimentation may be necessary to determine the most favourable process conditions.

Theoretically, recycle ratios of 100:1, 200:1 or even higher may be achieved. Practically, however, it should seldom be necessary to employ recycle ratios much in excess of 100:1. In general, factors such, as the aeration chamber design, the nature of the microbes involved, the nature of the desired product and the nature of the feed, will define what will be the upper limit for the recycle ratio in a given situation.

In accordance with the present invention, such as is illustrated in the Example, the recycle ratio will be in the range of from 30:1 to 120:1 preferably from 35:1 to 50:1.

The advantage of the recycle method in bringing about improved oxygen contact with the immobilized microorganisms may be seen by a consideration of the following illustrative flow rates in a continuous fermentation process. If the concentration of oxygen at saturation in a given feed material is 7 ppm and the feed rate to a conventional single pass reactor is 1 litre/hr, the oxygen delivery rate to the reactor is 7 mg/hr assuming that the feed has a specific gravity of 1.0. Using the same feed material and feed rate with a recyle · apparatus, where the aerated effluent has an oxygen concentration of 7 ppm and the recycle rate is 9 litre/hr (for a recycle ratio of 9:1), the amount of oxygen delivered to the reactor would be 70 mg/hr, a ten-fold increase.

The type of aeration chamber is not critical to the present invention. For instance, various of the conventional fermenter aeration systems or spargers may be used. Also, various normally-used construction materials, such as stainless steel and glass, may be employed.

While aeration in the aeration chamber will generally be accomplished by saturating the effluent contained therein with air, a pure oxygen stream may be used, as well as mixtures of oxygen with other gases.

Interestingly, it has been found that when an amount of oxygen sufficient to satisfy microbial demand is provided in a conventional single-pass reactor typically by means of relatively high flow rates, the effluent still does not contain the high concentrations of microbial cells which are obtained with the recycle apparatus in accordance with the present invention.

In operation, the recycle apparatus has a pool of medium, i.e. the liquid holdup, in the reactor containing suspended microbes which also contribute to cell production. An estimation of this contribution is not essential for the present purposes. In order to obtain a good estimate of the cells produced solely from the immobilized microbes, however, the contributions of cells produced due to the suspended microbes and microbes growing on the reactor wall must be allocated by estimating the fraction, $\phi$, of total cells in the reactor which are immobilized on the packing material or carrier. The specific cell output from the reactor may then be estimated by multiplying the observed output by the fraction $\phi$.

An estimate of $\phi$ requires a determination of cell loading on the carrier, i.e. the number of cells per unit weight of composite. Such a determination may be made by one or more known methods, such as the ATP-extraction method, elemental analysis (carbon and nitrogen, in particular) and ignition analysis. The ATP-extraction method is based upon the fact that the amount of adenosine triphosphate (ATP) contained in each bacterial cell is relatively constant. Thus, it is possible to estimate the number of immobilized cells per unit weight of composite by measuring the amount of ATP present per unit weight of composite. Breifly, the ATP contained in a known weight of composite is extracted with dimethylsulphoxide and the amount of ATP thus extracted is measured by chemiluminescence using, for example a DuPont Model 760 Biometer (E.I. DuPont de Nemours and Company, Wilmington, Delaware, U.S.A.). This is done by injecting an aliquot of the ATP extract into a cuvette containing a luciferein-luciferase mixture. A flash of light results, the intensity of which is measured and correlated with the concentration of ATP in the aliquot. The total amount of ATP extracted is then readily calculated from which the total number of cells may be estimated.

The measurement of cell loading by elemental analysis is as follows: The elemental composition of bacterial cells has been analysed and found to be reasonably constant. On this basis, the empirical representation of a bacterial cell is $C_5H_7NO_2$. Thus, the number of cells per gram of composite, where the carrier is inorganic, may be calculated if the percent carbon or nitrogen of the composite and the weight of a single cell are known. It is understood that the percent carbon or nitrogen value is converted to a decimal in order to represent the weight in grams of carbon or nitrogen per gram of composite. For example, if the percent carbon were 1.5, the value for grams of carbon per gram of composite would be 0.015. Thus, cell loading may be calculated as follows:

Carbon

$$\text{cell loading (cells/g)} = \text{g C/g composite} \times \frac{1}{60 \text{ g C/113 g cell mat.}} \times \frac{1}{\text{cell. wt., g cell mat./cell}} \qquad (1)$$

Nitrogen

$$\text{cell loading (cells/g)} = \text{g N/g composite} \times \frac{1}{14 \text{ g N/113 g cell mat.}} \times \frac{1}{\text{cell wt., g cell mat./cell}} \qquad (2)$$

A Perkin-Elmer (Norwalk, Connecticut, U.S.A.) Model 240 Elemental Analyzer may be used for the carbon and nitrogen analyses. Nitrogen content may also be determined by the Kjeldahl method.

As noted above, the elemental analysis method of estimating cell loading requires knowing the approximate weight of a single cell. For example, the cell weight of *E. coli* is about $5.6 \times 10^{-13}$ g (C. Lamanna, *et al.*, "Basic Bacteriology," Fourth Edition, The Williams & Wilkins Co., Baltimore, 1973, p. 67). Thus, for *E. coli*, equations (1) and (2) simplify to equations (3) and (4), respectively:

$$\text{cell loading (cells/g)} = (3.36 \times 10^{12} \text{ cells/g C}) \ (\text{g C/g composite}) \qquad (3)$$

$$\text{cell loading (cells/g)} = (1.44 \times 10^{13} \text{ cells/g N}) \ (\text{g N/g composite}) \qquad (4)$$

Where the carrier employed is inorganic, it is also possible to estimate cell loading by heating composite samples to 500°C for one hour and determining the percent loss in weight as a result of this ignition procedure (%LOI). If an estimate of the weight of a single cell is available, cell loading may be calculated as follows (the % LOI is converted to a decimal in order to represent the weight in grams of cell material per g of composite):

$$\text{cell loading (cells/g)} = \text{g cell mat./g composite} \times \frac{1}{\text{cell wt., g cell mat./cell}} \qquad (5)$$

For *E. coli*, equation (5) may be expressed as:

$$\text{cell loading (cells/g)} = (1.79 \times 10^{12} \text{ cells/g cell mat.}) \ (\text{g cell mat./g composite}) \qquad (6)$$

In calculating the value of φ, some combination of analytical methods for the determination of cell loading (such as those described above) is typically utilized. Furthermore, the measurement of effluent cell concentration may be used as an estimate of the suspended cell concentration in the liquid holdup volume. For convenience, the contribution of reactor wall growth is typically presumed to be negligible.

Thus, the following embodiments, expressed numerically, illustrate the calculation of φ. If the reactor of a recycle apparatus containing 2.34 g of carrier produces $21.56 \times 10^{10}$ cells/hr and if additional estimates are as follows, the value of φ is calculated as shown by equation (7):

| | |
|---|---|
| Estimated bed volume | =3.75 ml |
| Estimated liquid holdup | =40 ml |
| Estimated total volume | =43.75 ml |
| Estimated cell loading | =$5.1 \times 10^{10}$ cells/g |
| Estimated effluent cell concentration | =$5.33 \times 10^8$ cells/ml |

$$\phi = \frac{2.34 \text{ g} \times 5.1 \times 10^{10} \text{ cells/g}}{40 \text{ ml} \times 5.33 \times 10^8 \text{ cells/ml} + 2.34 \text{ g} \times 5.1 \times 10^{10} \text{ cells/g}} \qquad (7)$$

$$\phi = 0.848$$

Example

The feasibility of operating an immobilized microbe recycle apparatus was demonstrated using *E. coli* K12 as the microorganism. *E. coli* K12 cells were adsorbed on 18/25 mesh U.S. Standard Sieve (1.0/0.71 mm) porous cordierite particles. From 20 to 25 ml of an *E. coli* K12 cell suspension was contacted with from 2 to 3 g of the porous cordierite particles for from 3 to 4 hours at ambient temperature. Portions of the resulting composite were charged to several single-pass, fixed bed immobilized microbe reactors which then were operated at about 22°C under different flow rates; in each case the feed consisted of an aqueous solution of Bacto Nutrient Broth Dehydrate from Difco Laboratories. The results are summarized in Table 1 below.

TABLE 1

*E. coli* K12 production via single-pass immobilized microbe reactors

| Flow rate ml/hr | Mean cell conc. in effluent, $10^6$ cells/ml[a] | Estimated carrier weight, g | Estimated reactor volume, ml | Reactor productivity, $10^{10}$ cells/hr/ml bed volume |
|---|---|---|---|---|
| 100 | 21.00 | 0.80 | 1.3 | 0.16 |
| 1000 | 9.00 | 0.80 | 1.3 | 0.69 |
| 3882 | 5.76 | 0.63 | 1.0 | 2.24 |
| 4110 | 6.67 | 0.49 | 0.8 | 3.43 |
| 7950 | 4.34 | 0.60 | 1.0 | 3.45 |

[a]Measurements made under steady state conditions, typically after from 1 to 3 days.

At a feed rate of 100 ml/hr, the maximum cell concentration in the reactor effluent was determined by the viable plate count method to be $21 \times 10^6$ cells/ml. At this feed rate, it was found that only 6% of the oxygen originally dissolved in the feed remained in the effluent. Increasing the feed flow rate did not improve the maximum cell concentration in the effluent. Thus, at a feed flow rate of 1,000 ml/hr, the effluent cell concentration was found to be $9 \times 10^6$ cells/ml with only a 50% reduction in available oxygen. Additional runs demonstrated that further increases in the feed flow rate improved reactor productivity, but resulted in decreased effluent cell concentrations. As shown in Table 1, a feed flow rate of 4,110 ml/hr resulted in an effluent cell concentration of only about $7 \times 10^6$ cells/ml.

On the other hand, the recycle apparatus illustrated in Figure 2 brought about significantly improved results. In a manner similar to that described for the single-pass reactors *E. coli* K12 cells were adsorbed onto 18/25 mesh (1.0/0.71 mm) porous cordierite particles and placed in a reactor. Sterile nutrient broth was again used as feed. The recycle apparatus was then operated under varying feed flow rates and with varying amounts of composite. The data obtained are summarized in Table 2 below. It should be noted that, consistent with the data reported in Table 1, the total reactor productivities recorded in Table 2 include any contributions from suspended microbes and wall growth. For convenience and simplicity in reporting the data in Tables 1 and 2, such contributions were presumed to be minor. However, for a more rigorous analysis of the data, such contributions would be taken into account.

# 0 073 675

## TABLE 2
### *E. coli* K12 production with an immobilized microbe recycle reactor

| Mean effluent cell conc. $10^6$ cells/ml[a] | Recycle ratio | Feed rate, ml/hr | Carrier wt., g | $10^{10}$ cells/hr | Total reactor productivity, $10^{10}$ cells/hr/ml bed volume |
|---|---|---|---|---|---|
| 211 | 46 | 547 | 5.52 | 11.6 | 1.30 |
| 183 | 53 | 473 | 4.99 | 8.68 | 1.08 |
| 237 | 30 | 446 | 5.24 | 10.6 | 1.25 |
| 157 | 48 | 468 | 4.83 | 7.33 | 0.95 |
| 130 | 108 | 390 | 2.58 | 5.07 | 1.22 |
| 323 | 51 | 342 | 2.59 | 11.1 | 2.66 |
| 769 | 45 | 390 | 2.34 | 30.0 | 8.01 |
| 530 | 45 | 390 | 2.41 | 20.7 | 5.35 |
| 112 | 45 | 390 | 0.64 | 4.38 | 4.27 |
| 171 | 47 | 342 | 0.50 | 5.85 | 7.29 |
| 211 | 100 | 390 | 2.52 | 8.23 | 2.04 |
| 88.8 | 47 | 342 | 0.50 | 3.04 | 3.79 |
| 56.2 | 114 | 342 | 0.50 | 1.92 | 2.40 |
| 107 | 88 | 441 | 2.00 | 4.72 | 1.46 |
| 164 | 36 | 501 | 0.59 | 8.22 | 8.68 |
| 330 | 36 | 534 | 2.37 | 17.6 | 4.64 |

[a]Measurements made under steady state conditions, typically after from 1 to 3 days.

As may be seen, vastly improved cell production results from the use of an immobilized microbe recycle apparatus in accordance with the present invention. Under conditions giving comparable reactor productivities, the effluent cell concentrations for the recycle apparatus are about an order of magnitude higher. For example, with a recycle reactor containing 0.64 g of carrier, the mean cell concentration was $1.12 \times 10^8$ cells/ml. In contrast, even with about 0.8 g of carrier, the single-pass reactor effluent mean cell concentration was at best only $2.1 \times 10^7$ cells/ml (Table 1).

Early, preliminary runs of both the single-pass reactor and the recycle apparatus were evaluated using immobilized *E. coli* K12 cells for a period of about three days to determine the relationship of effluent cell concentration and time. In each case, the data (not reported) were plotted as the log of effluent cell concentration in cell/ml versus time in hours. Such plot is illustrated in Figure 4, wherein A is the curve obtained from the recycle apparatus data and B is the curve obtained from the single-pass reactor data. However, it should be noted that the data represented in Figure 4 were obtained before optimization studies had been carried out. Consequently, the steady state cell concentration of curve B is about $2.5 \times 10^6$ cells/ml, a value which is about 60% of the lowest value reported in Table 1. The steady state cell concentration of curve A, however is within the range of mean effluent cell concentrations reported in Table 2. Interestingly, recycle apparatus performance, whether measured by effluent cell concentration or reactor productivity, is clearly significantly less susceptible to operational parameters, such as feed flow rate, than is the single-pass reactor, at least for the growth of *E. coli* K12 cells within the ranges of variables studied. That is, even with the variations in amounts of carrier, feed flow rates and recycle ratios reported in Table 2, the mean effluent cell concentrations and total reactor productivities for the recycle apparatus varied only by a factor of about ten or less, while reactor productivity for the single-pass reactor varied by a factor of more than 20.

As mentioned above, the present invention has been described primarily with respect to the growth of aerobic microorganisms. However, the principles described herein are applicable to analogous situations

9

**0 073 675**

where cell growth is dependent primarily upon demand for one or more specific nutrients which cannot be supplied adequately in a batch or single-pass system. For example, the use of other nutrients or components which are sparingly soluble in the feed, such as nitrogen for nitrogen-fixing microbes; and vitamines desired in the growth medium, such as vitamines A, $D_4$ and E; would benefit from the application of the present invention.

**Claims**

1. A continuous fermentation apparatus characterised in that it comprises:
(A) an immobilized microbe reactor having an inlet and an outlet;
(B) a nutrient supply means;
(C) a first means connected to the said inlet for supplying feed thereto;
(D) a second means connecting the said outlet to the said nutrient supply means;
(E) a third means connecting the said nutrient supply means to the said first means and having a flow regulation means suitable for recycling a portion of the effluent from the said outlet to the said first means at a recycle ratio of nutrient-enriched effluent to feed of from 30:1 to 120:1; and
(F) a fourth means for withdrawing from the said continuous fermentation apparatus that portion of effluent from the said outlet which is not recycled to the said first means, which fourth means is connected to the said outlet, the said second means, the said nutrient supply means or the said third means at a point from the said nutrient supply means to and including the said flow regulation means.

2. An apparatus as claimed in claim 1 wherein the said fourth means is connected to the said nutrient supply means.

3. An apparatus as claimed in claim 1 wherein the said fourth means is connected to the said second means.

4. An apparatus as claimed in any of claims 1 to 3 wherein the nutrient supply means comprises a chamber for contacting a liquid with a gas.

5. An apparatus as claimed in claim 4 wherein the nutrient supply means comprises an aeration chamber.

6. A continuous fermentation process characterised in that it comprises:
(A) continuously passing a feed through an immobilized microbe reactor;
(B) continuously adding at least one productivity-limiting nutrient to at least a portion of the effluent from the said reactor;
(C) continuously recycling at least a portion of the nutrient-enriched effluent from (B) through the said reactor at a recycle ratio of nutrient-enriched effluent to feed of from 30:1 to 120:1, with the proviso that the volume ratio of effluent from the said reactor to recycled nutrient-enriched effluent is greater than unity; and
(D) continuously withdrawing for disposal or further processing that portion of effluent from the said reactor which is not recycled.

7. A process as claimed in claim 6 wherein the said at least one productivity-limiting nutrient is a gas which is sparingly soluble in the feed.

8. A process as claimed in claim 7 wherein the said gas is oxygen.

9. A process as claimed in claim 8 wherein the said oxygen is in the form of a free oxygen-containing gas.

10. A process as claimed in claim 9 wherein the said free-oxygen-containing gas is air.

**Patentansprüche**

1. Vorrichtung zur kontinuierlichen Fermentation, gekennzeichnet durch
(A) ein Reaktionsgefäß für immobilisierte Mikroben, das einen Einlaß und einen Auslaß besitzt;
(B) eine Nährstoffversorgungseinrichtung;
(C) eine erste Einrichtung, die mit dem Einlaß zur Nahrungszuführung verbunden ist;
(D) eine zweite Einrichtung, die den Auslaß mit der Nährstoffversorgungseinrichtung verbindet;
(E) eine dritte Einrichtung, die die Nährstoffversorgungseinrichtung mit der ersten Einrichtung verbindet und die eine Strömungsreguliereinrichtung aufweist, durch die ein Teil des Abflusses aus dem Auslaß zu der ersten Einrichtung mit einem Recyclingverhältnis des nährstoffangereicherten Abflusses zu Nahrung von 30:1 bis 120:1 zurückführbar ist; und
(F) eine vierte Einrichtung, durch die von der Vorrichtung zur kontinuierlichen Fermentation der Teil des Abflusses aus dem Auslaß entnehmbar ist, welcher nicht zu der ersten Einrichtung zurückgeführt wird, wobei die vierte Einrichtung mit dem Auslaß, der zweiten Einrichtung, der Nährstoffversorgungseinrichtung oder der dritten Einrichtung an einer die Strömungsreguliereinrichtung enthaltenden Stelle von der Nährstoffversorgungseinrichtung verbunden ist.

2. Vorrichtung nach Anspruch 1, dadurch gekennzeichnet, daß die vierte Einrichtung mit der Nährstoffversorgungseinrichtung verbunden ist.

10

3. Vorrichtung nach Anspruch 1, dadurch gekennzeichnet, daß die vierte Einrichtung mit der zweiten Einrichtung verbunden ist.

4. Vorrichtung nach einem der Ansprüche 1 bis 3, dadurch gekennzeichnet, daß die Nährstoffversorgungseinrichtung eine Kammer zum Kontaktieren einer Flüssigkeit mit einem Gas besitzt.

5. Vorrichtung nach Anspruch 4, dadurch gekennzeichnet, daß die Nährstoffversorgungseinrichtung eine Belüftungskammer besitzt.

6. Kontinuierliches Fermentationsverfahren gekennzeichnet durch, ·

(A) durch ein Reaktionsgefäß für immobilisierte Mikroben wird ständig eine Nahrung hindurchgeführt;

(B) wenigstens einem Teil des Ausflusses aus dem Reaktionsgefäß wird ständig wenigstens ein produktivitätsbegrenzender Nährstoff hinzugefügt;

(C) wenigstens ein Teil des nährstoffangereicherten Abflusses von (B) wird durch das Reaktionsgefäß mit einem Recyclingverhältnis von nährstoffangereicherten Abfluß zu Nahrung von 30:1 bis 120:1 kontinuierlich mit der Bedingung zurückgeführt, daß das Volumenverhältnis des Abflusses von dem Reaktionsgefäß zu den zurückgeführten nährstoffangereicherten Abfluss größer als 1 ist; und

(D) zur Ablage oder weiteren Verarbeitung wird ständig der Teil des Abflusses aus dem Reaktionsgefäß entnommen, der nicht zurückgeführt wird.

7. Verfahren nach Anspruch 6, dadurch gekennzeichnet, daß wenigstens ein produktionsbegrenzender Nährstoff ein Gas ist, das in der Nahrung schwer löslich ist.

8. Verfahren nach Anspruch 7, dadurch gekennzeichnet, daß das Gas Sauerstoff ist.

9. Verfahren nach Anspruch 8, dadurch gekennzeichnet, daß der Sauerstoff in Form eines freien Sauerstoff enthaltenden Gases vorliegt.

10. Verfahren nach Anspruch 9, dadurch gekennzeichnet, daß das freien Sauerstoff enthaltende Gas Luft ist.

**Revendications**

1. Un appareil de fermentation continue caractérisé en ce qu'il comprend:

(A) un réacteur à microbes immobilisés comportant une entrée et une sortie;

(B) un moyen d'alimentation en substance nutritive;

(C) un premier moyen relié à ladite entrée pour y introduire la charge d'alimentation;

(D) un deuxième moyen reliant ladite sortie audit moyen d'alimentation en substance nutritive;

(E) un troisième moyen reliant ledit moyen d'alimentation en substance nutritive audit premier moyen et comportant un moyen de régulation d'écoulement convenant pour recycler une partie de l'effluent provenant de ladite sortie dans ledit premier moyen à un rapport de recyclage de l'effluent enrichi en substance nutritive à la charge d'alimentation de 30:1 à 120:1; et

(F) un quatrième moyen destiné à soutirer dudit appareil de fermentation continue la partie d'effluent provenant de ladite sortie qui n'est pas recyclée vers ledit premier moyen, ce quatrième moyen étant relié à ladite sortie, audit second moyen, audit moyen d'alimentation en substance nutritive ou audit troisième moyen en un point situé à partir dudit moyen d'alimentation en substances nutritives jusqu'audit moyen de régulation d'écoulement inclus.

2. Un appareil selon la revendication 1, dans lequel ledit quatrième moyen est relié audit moyen d'alimentation en substance nutritive.

3. Un appareil selon la revendication 1, dans lequel ledit quatrième moyen est relié audit deuxième moyen.

4. Un appareil selon l'une quelconque des revendications 1 à 3 dans lequel le moyen d'alimentation en substance nutritive comprend une chambre destinée à mettre un liquide en contact avec un gaz.

5. Un appareil selon la revendication 4, dans lequel le moyen d'alimentation en substance nutritive comprend une chambre d'aération.

6. Un procédé de fermentation continue, caractérisé en ce qu'il consiste:

(A) à faire passer en continu une charge d'alimentation à travers un réacteur à microbes immobilisés;

(B) à ajouter en continu au moins une substance nutritive limitant la productivité à au moins une partie de l'effluent provenant dudit réacteur;

(C) à recycler en continu au moins une partie de l'effluent enrichi en substance nutritive provenant de (B) à travers ledit réacteur à une rapport de recyclage de l'effluent enrichi en substance nutritive à la charge d'alimentation de 30:1 à 1201, à condition que le rapport en volume de l'effluent dudit réacteur à l'effluent enrichi en substance nutritive recyclé soit supérieur à l'unité; et

(D) à soutirer en continu pour la rejeter ou la traiter supplémentairement la partie de l'effluent dudit réacteur qui n'est pas recyclée.

7. Un procédé selon la revendication 6, dans lequel la substance nutritive limitant la productivité est un gaz qui est peu soluble dans la charge d'alimentation.

8. Procédé selon la revendication 7, dans lequel ledit gaz est l'oxygène.

9. Procédé selon la revendication 8, dans lequel l'oxygène est sous la forme d'un gaz contenant de l'oxygène libre.

10. Procédé selon la revendication 9, dans lequel le gaz contenant de l'oxygène libre est l'air.

Fig. 1

Fig. 2

Fig. 3

Fig. 4